# FASCICULE DE BREVET EUROPEEN

(11) **EP 3 525 836 B1**
(45) Date de publication et mention de la délivrance du brevet: **21.10.2020**
(21) Numéro de dépôt: 16794534.4
(22) Date de dépôt: 24.10.2016
(51) Int. Cl.: A61M 1/02, G06K 19/077, G06K 7/10

(54) **PROCÉDÉ DE GESTION D'AU MOINS UN CONTENEUR ET PROCÉDÉS ET DISPOSITIFS ASSOCIÉS**
VERFAHREN ZUR VERWALTUNG VON MINDESTENS EINEM BEHÄLTER UND ZUGEHÖRIGE VERFAHREN UND VORRICHTUNGEN
METHOD FOR MANAGING AT LEAST ONE CONTAINER AND ASSOCIATED METHODS AND DEVICES

(30) Priorité: 11.10.2016 FR 1659827
(43) Date de publication de la demande: 21.08.2019
(73) Titulaire: Biolog-id, 92100 Boulogne-Billancourt (FR)
(72) Inventeur: MONGRENIER, Jean-Claude, 78000 Versailles (FR)
(74) Mandataire: Lavoix
(86) Numéro de dépôt international: PCT/EP2016/075513
(87) Numéro de publication internationale: WO 2018/068872

(56) Documents cités:
- EP-A1- 3 076 339
- WO-A1-2016/065113
- FR-A1- 2 825 637
- US-A1- 2009 189 816

## Description

La présente invention concerne un procédé de gestion d'au moins un conteneur contenant un produit biologique. La présente invention se rapporte également à un procédé de transmission, un procédé de suivi, un dispositif de gestion et à un ensemble.

WO 2016/065113 A1 divulgue un procédé et un dispositif de gestion d'au moins un conteneur (poche de sang) contenant un produit biologique. Chacun des conteneurs comprend une étiquette RFID opérant à une fréquence de 13.56 MHz. L'étiquette RFID stocke des informations concernant la poche du sang dans sa mémoire. Les conteneurs sont stockés dans un réfrigérateur. Un lecteur ayant une antenne lit les informations stockées sur les étiquettes RFID afin de gérer le contenu du réfrigérateur.

EP 3 076 339 A1 divulgue un transpondeur (étiquette) à double fréquences comprenant une première antenne HF qui est apte à fonctionner à 13.56 MHz utilisant le protocole NFC pour une portée de communication courte (moins de 10 cm) et une seconde antenne UHF qui est apte à fonctionner à 900 MHz utilisant le protocole EPC Class-1 Gen 2. En outre, chacune des antennes est branchée à un modulateur/démodulateur, une unité logique et une mémoire. De plus, une partie de l'espace mémoire utilisée en mode HF ne correspond pas à la partie de l'espace mémoire utilisée en mode UHF.

La présente invention se rapporte au domaine de la logistique de conteneurs.

De tels conteneurs sont par exemple des poches contenant des produits biologiques tels que des produits sanguins (poches de sang primaire, de plasma, de plaquette, de globules rouges...) ou des produits d'ingénierie cellulaire (cellules souches...), ou encore des poches de médicaments telles que des poches de chimiothérapie.

La logistique de tels conteneurs impose non seulement d'assurer une exposition minimale des conteneurs à une température relativement élevée mais aussi de garantir la confidentialité de certaines informations.

En effet, pour certains produits biologiques, la législation impose un délai maximal d'exposition des conteneurs à une température supérieure à - 4°C. Ce délai maximal est fixé en Europe à 2 heures 30 par une directive.

Concernant la confidentialité, à titre d'exemple, le patient à qui est destiné le conteneur en cas de transfusion est une information non publique. Il convient donc de développer une méthode permettant d'empêcher une personne non habilitée de récupérer l'ensemble des informations relatives aux conteneurs facilement.

Il existe donc un besoin pour un procédé de gestion d'au moins un conteneur contenant un produit biologique permettant de traiter rapidement un ensemble de conteneurs tout en permettant une protection des données associés au conteneur.

Selon l'invention, ce but est atteint par un procédé de gestion d'au moins un conteneur contenant un produit biologique selon la revendication 1.

Suivant des modes de réalisation particuliers, le procédé de gestion comprend une ou plusieurs des caractéristiques suivantes, prise(s) isolément ou suivant toutes les combinaisons techniquement possibles :
- pendant la lecture HF, le deuxième lecteur lit une unique étiquette à la fois.
- l'étiquette communique une première partie des informations pendant la lecture UHF et une deuxième partie des informations pendant la lecture HF, la deuxième partie n'étant pas incluse dans la première partie.
- la première partie des informations est dépourvue d'informations relatives à la provenance du produit biologique.
- la communication entre la puce et le lecteur selon le mode HF est mise en œuvre avec le protocole NFC.

L'invention concerne aussi un procédé de transmission d'informations d'un conteneur mère à une pluralité de conteneurs filles, le procédé comprenant les étapes suivantes : fourniture d'un conteneur mère contenant un produit biologique, gestion du conteneur mère par mise en œuvre d'un procédé de gestion appliqué au conteneur mère tel que défini précédemment pour obtenir des informations relatives au conteneur mère, centrifugation du conteneur mère par une centrifugeuse pourvue d'un lecteur UHF, le lecteur UHF détectant la présence du conteneur mère lors de la centrifugation par communication avec l'étiquette du conteneur mère selon le mode UHF, séparation de composants du produit biologique en une pluralité de conteneurs filles, et écriture sur chaque mémoire des étiquettes des conteneurs filles des informations relatives au conteneur mère et des informations relatives à la centrifugation grâce à une communication selon le mode HF.

L'invention concerne en outre un procédé de suivi de conteneurs dans une enceinte à atmosphère contrôlée, comprenant les étapes suivantes : fourniture d'une enceinte à atmosphère contrôlée présentant une pluralité de compartiments délimitant chacun un volume intérieur, l'enceinte étant équipée d'au moins un lecteur UHF, des conteneurs étant placés dans divers emplacements déterminés, gestion des conteneurs par la mise en œuvre du procédé de gestion appliqué aux conteneurs tel que défini précédemment, comprenant la lecture de l'ensemble des étiquettes des conteneurs dans chaque compartiment par le lecteur UHF correspondant, et suivi d'un emplacement pour chaque conteneur présent dans l'enceinte, l'emplacement correspondant au lecteur UHF correspondant.

L'invention concerne par ailleurs un dispositif de gestion d'au moins un conteneur selon la revendication 8.

L'invention concerne aussi un ensemble selon la revendication 9.

D'autres caractéristiques et avantages de l'invention apparaîtront à la lecture de la description qui suit de modes de réalisation de l'invention, donnée à titre d'exemple uniquement et en référence aux dessins qui sont :
- figure 1, une représentation schématique d'un dispositif de gestion, et
- figure 2, une représentation schématique d'une enceinte à atmosphère contrôlée et
- figure 3, une représentation schématique d'une centrifugeuse.

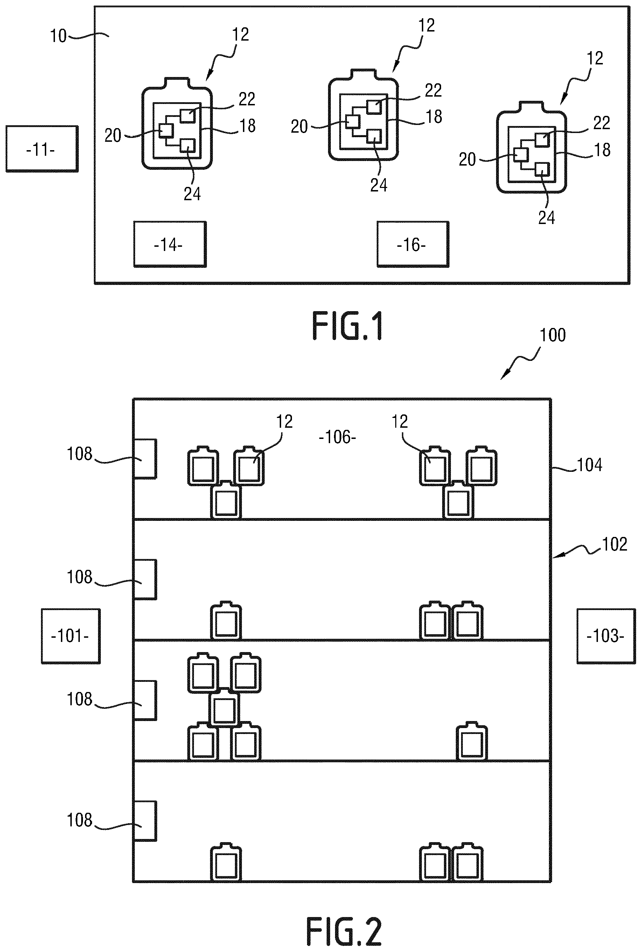

Un dispositif de gestion 10, simplement noté dispositif 10 dans la suite, est représenté sur la figure 1.

Le dispositif 10 comporte un calculateur 11, trois conteneurs 12, un premier lecteur 14 et un deuxième lecteur 16.

Selon d'autres modes de réalisation, le dispositif 10 comporte un nombre différent de conteneurs 12 et/ou un nombre différent de lecteurs 14, 16.

Dans tous les modes de réalisation, le dispositif 10 comporte au moins un conteneur 12 et un lecteur 14, 16.

Le calculateur 11 est propre à mettre en œuvre un procédé de gestion d'au moins un conteneur 12.

En variante, le calculateur 11 est une unité de traitement interagissant avec un produit programme d'ordinateur pour mettre en œuvre des instructions contenues dans le produit programme d'ordinateur et permettant de la mise en œuvre d'un procédé de gestion d'au moins un conteneur 12.

Par « gestion », il est entendu aussi bien une problématique d'inventaire (nombre de conteneurs 12), qu'une problématique d'identification, c'est-à-dire d'association d'un conteneur 12 à des informations spécifiques, notamment relative au contenu du conteneur 12.

Chaque conteneur 12 comporte des produits biologiques.

De manière générale, le conteneur 12 est tout type de poche destinée à contenir des produits dont l'utilisation est conditionnée par des contraintes de stockage strictes.

Dans un mode de réalisation, les conteneurs 12 sont des poches contenant des produits biologiques tels que des produits sanguins (poches de sang primaire, de plasma, de plaquettes, de globules rouges...) ou des produits d'ingénierie cellulaire (cellules humaines ou animales, notamment cellules souches humaines ou animales, produits issus de cellules humaines ou animales).

Dans un autre mode de réalisation, les conteneurs 12 sont des poches de médicaments ou de préparations thérapeutiques contenant un ou plusieurs principes actifs ou médicaments, telles que des poches de chimiothérapie (contenant généralement un soluté et un ou plusieurs principes actifs de chimiothérapie).

De manière plus générale, le conteneur 12 est propre à contenir tout produit destiné à être perfusé à un patient (humain ou animal).

Selon l'exemple considéré, chaque conteneur 12 est une poche destinée, dans le cas présent, à contenir des produits sanguins.

De façon connue, un tel conteneur 12 est un contenant étanche de produit en matière plastique respirante permettant le métabolisme, du type PVC (polychlorure de vinyle), polycarbonate ou PEG (polyéthylène glycol).

Le conteneur 16 comporte plusieurs faces et est pourvu d'une étiquette 18.

Selon l'exemple représenté, l'étiquette 18 est une étiquette adhésive qui est collée sur une face du conteneur 12.

L'étiquette 18 est une puce de communication sans fil.

L'étiquette 18 comporte une antenne radiofréquence 20, une mémoire 22 et un microprocesseur 24.

L'étiquette 18 est une étiquette propre à fonctionner selon deux gammes de fréquences distinctes. En ce sens, la puce de communication sans fil peut être qualifiée de puce bi-fréquence.

D'une part, l'étiquette 18 est apte à communiquer selon un premier mode de fonctionnement appelé mode UHF. L'acronyme « UHF » renvoie à la terminologie française d'ultra haute fréquence.

Dans un tel mode, l'étiquette 18 est apte à émettre ou à recevoir un signal ayant une fréquence comprise entre 300 MHz et 3 000 MHz.

D'autre part, l'étiquette 18 est apte à communiquer selon un deuxième mode de fonctionnement appelé mode HF. L'acronyme « HF » renvoie à la terminologie française d'haute fréquence.

Dans un tel mode, l'étiquette 18 est apte à émettre ou à recevoir un signal ayant une fréquence comprise entre 3 MHz et 30 MHz.

Chaque étiquette 18 est associée à un identifiant unique.

L'identifiant unique est mémorisé dans la mémoire 20.

La mémoire 22 comprend, par exemple, les informations suivantes : l'identifiant unique, un numéro de commande, la nature du produit contenu dans le conteneur, une catégorie du produit, par exemple, la catégorie immunologique, le nom ou un identifiant d'un patient à perfuser et/ou une donnée de contrôle.

L'antenne 20 permet d'envoyer ou de recevoir des signaux.

Le microprocesseur 24 est apte à communiquer selon un premier protocole et un deuxième protocole distinct du premier protocole.

Le premier protocole est, par exemple, conforme aux standards ISO14443 ou ISO15693.

Le deuxième protocole est, par exemple, conforme aux standards ISO18000-6C aussi connu sous le nom d'EPC1Gen2.

Le microprocesseur 24 est, en outre, apte à mettre en œuvre un certain nombre de commandes spéciales dues à la gestion des deux protocoles.

La description qui vient d'être faite d'un conteneur 12 s'applique pour tous les conteneurs 12.

Le premier lecteur 14 est un lecteur apte à communiquer avec une étiquette 18 selon le mode UHF.

Le premier lecteur 14 présente une portée de communication

La portée de communication du premier lecteur 14 est de préférence supérieure à 1 mètre (m), plus particulièrement comprise entre 5 mètres et 10 mètres.

Le deuxième lecteur 16 est un lecteur apte à communiquer avec une étiquette 18 selon le mode HF.

La communication entre la puce et le lecteur selon le mode HF est mise en œuvre avec un protocole de la norme NFC. Par l'acronyme « NFC », il est étendu le terme de « near field communication » qui signifie « communication de champ proche ».

Selon un exemple particulier, le protocole de communication est conforme aux standards ISO 14443, ISO 15693 ou ISO 18000-3m.

Le deuxième lecteur 16 présente une portée de communication

La portée de communication du deuxième lecteur 16 est de préférence inférieure à 20 centimètres (cm).

L'espace entre l'étiquette 18 et le deuxième lecteur 16 correspond ainsi à une distance de détection.

En outre, le deuxième lecteur 16 est propre à alimenter l'étiquette 18 en mode HF.

Le fonctionnement du dispositif 10 est maintenant décrit en référence à un exemple de mise en œuvre d'un procédé de gestion d'au moins un conteneur 12 contenant un produit biologique.

Le procédé de gestion comporte une étape de fourniture d'un conteneur 12 munie d'une étiquette 18.

Le procédé de gestion comporte aussi une étape de lecture d'au moins une information comprise dans la mémoire de l'étiquette par un lecteur 14 ou 16.

Pendant la lecture, l'étiquette 18 communique avec le lecteur 14 ou 16 selon le mode UHF ou le mode HF.

Selon un exemple, le procédé de gestion comprend une lecture dite UHF.

Un premier lecteur 14 lit l'ensemble des étiquettes 18 présentes dans la portée du premier lecteur 14, les étiquettes communiquant avec ledit lecteur selon le mode UHF.

Pendant la lecture UHF, l'étiquette 18 communique une première partie des informations de la mémoire comprenant, par exemple, l'identifiant unique.

Cela permet, par exemple, d'identifier rapidement l'ensemble des conteneurs 12 à une distance donnée.

Alternativement ou en complément, le procédé de gestion comprend une lecture dite HF.

Le deuxième lecteur 16 lit une unique étiquette 18 à la fois, l'étiquette 18 communiquant avec le lecteur selon le mode HF.

Pendant la lecture HF, l'étiquette 18 communique une deuxième partie des informations de la mémoire 22 de l'étiquette 18.

La deuxième partie des informations de la mémoire 22 n'est pas incluse dans la première partie, c'est-à-dire qu'il y a au moins une information comprise dans la mémoire apte à être communiquée par lecture HF mais pas par lecture UHF.

Les informations non incluses dans la première partie de la mémoire 22 sont, par exemple, l'identité d'un patient à perfuser, la destination d'un conteneur 12 lors d'un transfert.

La lecture UHF et la lecture HF sont, par exemple, réalisées simultanément.

Un tel procédé de gestion permet de distinguer un conteneur 12 donné d'un autre conteneur 12, qui ne contient pas nécessairement le même produit. Plus particulièrement, cela permet à un professionnel médical de vérifier l'identifiant du conteneur 12 et la nature du produit contenu dans le conteneur 12 avant de perfuser un patient avec le conteneur 12 en lisant l'étiquette dudit conteneur 12.

Le procédé de gestion permet notamment de sécuriser des informations privées, de sorte qu'il ne soit pas facilement possible de récupérer rapidement les informations privées d'une pluralité de conteneurs. Une personne souhaitant connaître ces informations privées doit avoir un accès proche aux conteneurs 12 et doit lire par mode HF chaque étiquette 18.

L'utilisation d'une puce bi-fréquence dans la gestion de conteneurs 12 contenant un produit biologique permet ainsi d'assurer un suivi des conteneurs23 permettant à la fois une lecture rapide d'un ensemble d'étiquettes et une protection des données privées.

De plus, cela permet notamment un suivi d'emplacement rapide et une localisation plus précise. Le personnel médical connaît donc une localisation des conteneurs souhaités pour gagner du temps et maintenir les conditions de conservation des conteneurs 12.

Une telle idée est également utilisé dans l'ensemble 100 représenté à la figure 2.

L'ensemble 100 comporte un calculateur 101, une enceinte 102 et un lecteur 103.

Le calculateur 101 est propre à mettre en œuvre des procédés, comme un procédé de suivi de conteneurs 12 ou de transmission d'informations entre conteneurs 12.

L'enceinte 102 est une enceinte 102 destinée à accueillir des conteneurs 12 dans une atmosphère contrôlée.

Une atmosphère est qualifiée de « contrôlée » lorsqu'au moins un paramètre de l'atmosphère est contrôlée régulièrement. Le paramètre est, selon les cas, la température, la pression ou l'humidité.

Pour cela, l'enceinte 102 est usuellement appelée enceinte à atmosphère contrôlée.

L'enceinte 102 est, par exemple, un réfrigérateur ou un agitateur.

L'enceinte 102 à atmosphère contrôlée présente une pluralité de compartiments 104 délimitant chacun un volume intérieur 106.

Les compartiments 102 sont aptes à recevoir des conteneurs 12 dans le volume intérieur 106.

Dans un premier mode de réalisation, chaque compartiment 104 comporte des parois blindées de sorte que les signaux ayant une fréquence comprise entre 3 MHz et 3 000 MHz ne parviennent pas à entrer ou sortir du compartiment 104.

Chaque paroi du compartiment 104 est, par exemple, réalisé en métal non ferromagnétique, notamment en aluminium, cuivre ou en acier inoxydable notamment selon la norme européenne EN 10027 X5CrNi18-10 1.4301 correspondant à la norme américaine AISI 304.

Chaque paroi présente une épaisseur supérieure à trois épaisseurs de peau. L'épaisseur est déterminée selon la nature du matériau de la paroi et la fréquence des signaux que l'on souhaite bloquer.

Dans un mode de réalisation, chaque paroi est faite en aluminium avec une épaisseur égale à 150 µm. Cela permet notamment de bloquer les signaux ayant une fréquence égale à 3 MHz. En outre, une épaisseur de 66 µm d'aluminium est apte à bloquer les signaux ayant une fréquence égale à 13,56 MHz.

Alternativement, chaque paroi est faite en cuivre avec une épaisseur égale à 100 µm pour bloquer les signaux ayant une fréquence égale à 3 MHz ou une épaisseur égale à 50 µm pour bloquer les signaux ayant une fréquence égale à 13,56 MHz.

Alternativement, chaque paroi est faite en acier inoxydable avec une épaisseur égale à 700 µm pour bloquer les signaux ayant une fréquence égale à 3 MHz ou à une épaisseur égale 360 µm pour bloquer les signaux ayant une fréquence égale à 13,56 MHz.

En variante, les compartiments sont espacés les uns des autres, les signaux ayant une fréquence comprise entre 3 MHz et 3 000 MHz n'étant pas aptes de se propager d'un compartiment 104 à un autre compartiment 104.

L'espace entre deux compartiments 104 est alors suffisamment grand pour que l'atténuation des signaux à une fréquence comprise entre 3 MHz et 3 000 MHz lors de leur propagation entre deux compartiments 104 permette de négliger la possibilité de détecter les signaux en provenance d'un autre compartiment 104.

Chaque compartiment 104 est équipé d'un lecteur 108 propre distinct du lecteur HF 101.

Chaque lecteur 108 d'un compartiment 104 est apte à recevoir un signal ayant une fréquence comprise entre 300 MHz et 3 000 MHz.

Dans la suite, chaque lecteur 108 est dit lecteur UHF 108.

Dans un deuxième mode de réalisation, un signal ayant une fréquence comprise entre 3 MHz et 3 000 MHz est apte à circuler dans l'enceinte 102. L'enceinte 102 présente alors, par exemple, un unique lecteur UHF.

Le lecteur 103 est un lecteur similaire au deuxième lecteur 16.

Aussi, dans la suite, le lecteur 103 est appelé lecteur HF 103.

Le fonctionnement de l'ensemble 10 est maintenant décrit en référence à un exemple de mise en œuvre d'un procédé de suivi de conteneurs 12 dans l'enceinte 102.

Le procédé de suivi comporte une étape de de fourniture de l'enceinte 102, des conteneurs 12 étant placés dans divers emplacements déterminés.

Le procédé de suivi comporte alors une mise en œuvre de la gestion des conteneurs 12 par la mise en œuvre du procédé de gestion précédemment décrit appliqué aux conteneurs 12.

Notamment, l'étape de gestion des conteneurs 12 comprend une lecture de l'ensemble des étiquettes 18 des conteneurs 12 dans chaque compartiment 104 par le lecteur UHF 108 correspondant.

Le lecteur UHF 108 a une portée comprenant l'ensemble du volume intérieur 106 pouvant être occupé par des conteneurs 12.

Ainsi, pendant la gestion, le ou les lecteurs UHF 108 détectent toutes les étiquettes 18 des conteneurs 12 dans l'enceinte 102.

Le procédé de suivi comporte également une étape de suivi de l'emplacement de chaque conteneur 12.

La nature du suivi dépend des cas.

A titre d'exemple, le ou les lecteurs UHF 108 et le lecteur HF 103 de l'enceinte 102 transmettent au calculateur 101 des informations relatives aux conteneurs 12 détectés, par exemple, l'identifiant des conteneurs 12 et la nature et éventuellement la catégorie de leur contenu.

Ainsi, le calculateur 101 est apte à calculer et afficher le nombre de conteneurs 12 restants contenant un contenu d'une certaine nature et/ou catégorie. Dans le cas des poches sanguines, le calculateur 101 affiche le nombre de conteneurs par groupe sanguin, voir par rhésus.

Le calculateur 101 est, par exemple, apte à envoyer une alerte lorsque le nombre de conteneurs contenant un contenu d'une certaine nature et/ou catégorie est sous un seuil. Par exemple, lorsque le nombre de conteneurs de sang de groupe O est inférieur à un seuil détermine, le calculateur 101 émet une alerte, le sang de groupe O servant de groupe de sang d'urgence et étant a priori compatible avec l'ensemble des patients.

Selon un autre exemple, le suivi de l'emplacement permet d'obtenir la localisation du conteneur 12.

Selon un mode de réalisation non représenté, chaque compartiment 104 comporte, en outre, une antenne HF.

Le calculateur 101 est apte à savoir si la puce est détectée par une antenne HF ou une antenne UHF.

Pour chaque conteneur 12, au moins une antenne HF est activée à tour de rôle jusqu'à ce qu'une antenne HF détecte la présence de l'étiquette 18 correspondante.

Plus particulièrement, pour accélérer la localisation, la détection est réalisée par dichotomie : une première moitié des antennes HF est d'abord activée, si l'étiquette 18 est détectée par la première moitié, alors il est activé une moitié de la première moitié des antennes HF, sinon il est activé une moitié de la deuxième moitié des antennes HF. Il est ainsi continué jusqu'à ce qu'une seule antenne HF soit activée et que l'on puisse en déduire quelle antenne est apte à détecter la présence de l'étiquette.

La localisation du conteneur 12 correspond à l'antenne HF détectant l'étiquette.

La localisation du conteneur 12 est comprise dans l'emplacement associé à ladite antenne HF.

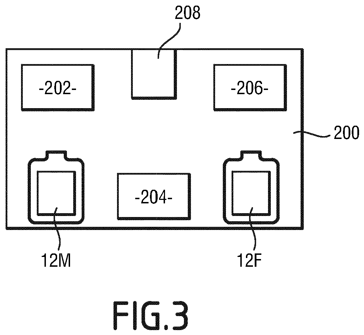

La figure 3 décrit une centrifugeuse 200 pourvue d'un calculateur 202, d'un lecteur UHF 204, une unité de centrifugation 206 et un moyen d'écriture 208.

Le calculateur 202 est propre à mettre en œuvre un procédé de transmission d'informations d'un conteneur mère 12M à une pluralité de conteneurs filles 12F.

Chaque conteneur mère 12M ou conteneur fille 12F comporte une étiquette 18 comme précédemment décrite.

Le lecteur UHF 204 est similaire aux lecteurs UHF présentés précédemment.

L'unité de centrifugation 206 assure la formation des conteneurs filles 12F à partir du conteneur mère 12M.

Le moyen d'écriture 208 est un organe propre à écrire sur des étiquettes 18

Le fonctionnement de la centrifugeuse 200 est maintenant décrit en référence à un exemple de mise en œuvre d'un procédé de transmission d'informations d'un conteneur mère 12M à une pluralité de conteneurs filles 12F.

Le procédé de transmission comporte une étape de fourniture d'un conteneur mère 12M contenant un produit biologique.

Le procédé de transmission comporte une étape de gestion du conteneur mère 12M.

L'étape de gestion du conteneur 12 mère correspond à la mise en œuvre du procédé de gestion décrit précédemment appliqué au conteneur mère 12M.

Le procédé de gestion appliqué au conteneur mère 12M comprend, par exemple, une étape de lecture HF.

L'étape de gestion permet d'obtenir des informations relatives au conteneur mère 12M.

Le procédé de transmission comporte alors une étape de centrifugation du conteneur mère 12M.

Pendant la centrifugation, le lecteur UHF 204 détecte la présence du conteneur mère 12F par communication avec l'étiquette du conteneur mère 12F selon le mode UHF.

L'utilisation de l'UHF permet notamment de suivre le conteneur mère 12M dans la centrifugeuse, y compris pendant la rotation. Ainsi, le lecteur UHF 204 est apte à vérifier que le conteneur 12 est resté dans l'unité de centrifugation 206 pendant toute l'étape de centrifugation.

Le procédé de transmission comprend ensuite une étape de séparation de composants du produit biologique en une pluralité de conteneurs filles 12F.

Le procédé de transmission comporte enfin une étape d'écriture sur chaque mémoire 22 des étiquettes 18 des conteneurs filles 12F.

Dans chaque mémoire 22 des étiquettes 18 des conteneurs filles 12F, des informations relatives au conteneur mère 12M et des informations relatives à la centrifugation sont écrites grâce à une communication selon le mode HF.

Cela permet un suivi entre le conteneur mère 12M et les conteneurs filles 12F.

Plus généralement, dans chacun des modes de réalisation, l'utilisation d'une étiquette bi-fréquence dans la gestion de conteneurs 12 contenant un produit biologique permet ainsi d'assurer un suivi des conteneurs 12 permettant à la fois une lecture rapide d'un ensemble d'étiquettes 18 et une protection des données privées.

De manière plus spécifique, l'utilisation d'une étiquette bi-fréquence offre à l'utilisateur l'avantage de bénéficier selon les contextes des avantages d'une technique par rapport à une autre. Dans certaines utilisations, la rapidité du mode UHF est à privilégier alors que, pour d'autres, c'est la précision du mode HF qu'il convient de privilégier.

## Revendications

1. Procédé de gestion d'au moins un conteneur (12) contenant un produit biologique, comprenant une étape de fourniture d'au moins un conteneur (12), chaque conteneur (12) étant pourvu d'une étiquette (18), l'étiquette (18) étant une puce de communication sans fil apte à communiquer selon un premier mode de fonctionnement dit mode UHF et selon un deuxième mode de fonctionnement dit mode HF, la puce étant apte à émettre ou recevoir un signal ayant une fréquence comprise entre 300 MHz et 3 000 MHz dans le mode UHF et apte à émettre ou recevoir un signal ayant une fréquence comprise entre 3 MHz et 30 MHz dans le mode HF, l'étiquette (18) comprenant en outre une mémoire (22) comprenant des informations, la puce étant apte à communiquer avec un premier lecteur (14) selon le mode UHF et avec un deuxième lecteur (16) selon le mode HF,
le procédé comprenant en outre les étapes suivantes :
- lecture dite UHF, dans laquelle le premier lecteur (14) présentant une portée donnée lit l'ensemble des étiquettes (18) présentes dans la portée, les étiquettes (18) communiquant avec le premier lecteur (14) selon le mode UHF, et
- lecture dite HF d'au moins une information privée non accessible par la lecture UHF, dans laquelle le deuxième lecteur (16) lit une étiquette (18), l'étiquette (18) communiquant avec le deuxième lecteur (16) selon le mode HF.

2. Procédé de gestion selon la revendication 1, dans lequel, pendant la lecture HF, le deuxième lecteur (16) lit une unique étiquette (18) à la fois.

3. Procédé de gestion selon la revendication 1 ou 2, dans lequel l'étiquette (18) communique une première partie des informations pendant la lecture UHF et une deuxième partie des informations pendant la lecture HF, la deuxième partie n'étant pas incluse dans la première partie.

4. Procédé de gestion selon la revendication 3, dans lequel la première partie des informations est dépourvue d'informations relatives à la provenance du produit biologique.

5. Procédé de gestion selon l'une quelconque des revendications 1 à 4, dans lequel la communication entre la puce et le lecteur (14, 16) selon le mode HF est mise en œuvre avec le protocole NFC.

6. Procédé de transmission d'informations d'un conteneur mère (12M) à une pluralité de conteneurs filles (12F), le procédé comprenant les étapes suivantes :
- fourniture d'un conteneur mère (12M) contenant un produit biologique,
- gestion du conteneur mère par mise en œuvre d'un procédé de gestion appliqué au conteneur mère (12M) selon l'une quelconque des revendications 1 à 5 pour obtenir des informations relatives au conteneur mère (12M),
- centrifugation du conteneur mère (12M) par une centrifugeuse pourvue d'un lecteur UHF, le lecteur UHF détectant la présence du conteneur mère (12M) lors de la centrifugation par communication avec l'étiquette (18) du conteneur mère (12M) selon le mode UHF,
- séparation de composants du produit biologique en une pluralité de conteneurs filles (12F), et
- écriture sur chaque mémoire (22) des étiquettes (18) des conteneurs filles (12F) des informations relatives au conteneur mère (12M) et des informations relatives à la centrifugation grâce à une communication selon le mode HF.

7. Procédé de suivi de conteneurs (12) dans une enceinte (102) à atmosphère contrôlée, comprenant les étapes suivantes :
- fourniture d'une enceinte (102) à atmosphère contrôlée présentant une pluralité de compartiments (104) délimitant chacun un volume intérieur (106), l'enceinte (102) étant équipée d'au moins un lecteur UHF (108), des conteneurs (12) étant placés dans divers emplacements déterminés,
- gestion des conteneurs (12) par la mise en œuvre du procédé de gestion appliqué aux conteneurs (12) selon l'une quelconque des revendications 1 à 5, comprenant la lecture de l'ensemble des étiquettes (18) des conteneurs (12) dans chaque compartiment (104) par le lecteur UHF (108) correspondant, et
- suivi d'un emplacement pour chaque conteneur (12) présent dans l'enceinte (102), l'emplacement correspondant au lecteur UHF (108) correspondant.

8. Dispositif (10) de gestion d'au moins un conteneur (12) contenant un produit biologique, le dispositif comprenant un lecteur (14, 16) et au moins un conteneur (12), le conteneur (12) étant pourvu d'une étiquette (18), l'étiquette (18) étant une puce de communication sans fil apte à communiquer selon un premier mode de fonctionnement dit mode UHF et selon un deuxième mode de fonctionnement dit HF, la puce étant apte à émettre ou recevoir un signal ayant une fréquence comprise entre 300 MHz et 3 000 MHz dans le mode UHF et apte à émettre ou recevoir un signal ayant une fréquence comprise entre 3 MHz et 30 MHz dans le mode HF, l'étiquette (18) comprenant en outre une mémoire (22) comprenant des informations, la puce étant apte à communiquer avec le lecteur selon le mode UHF et le mode HF (14, 16),
dans lequel l'étiquette (18) est apte à communiquer avec le lecteur (14, 16) une première partie des informations selon le mode UHF et une deuxième partie des informations comprenant au moins une information privée non accessible par lecture UHF selon le mode HF.

9. Ensemble (100) comprenant un lecteur dit HF (103), apte à recevoir un signal ayant une fréquence comprise entre 3 MHz et 30 MHz, et une enceinte (102) à atmosphère contrôlée présentant une pluralité de compartiments (104) délimitant chacun un volume intérieur (106), chaque compartiment (104) étant équipé d'un lecteur dit UHF (108) apte à recevoir un signal ayant une fréquence comprise entre 300 MHz et 3 000 MHz, des conteneurs (12) étant placés dans divers emplacements déterminés de l'enceinte (102),
chaque conteneur (12) étant pourvu d'une étiquette (18), l'étiquette (18) étant une puce de communication sans fil apte à communiquer selon un premier mode de fonctionnement dit mode UHF et selon un deuxième mode de fonctionnement dit mode HF, la puce étant apte à émettre ou recevoir un signal ayant une fréquence comprise entre 300 MHz et 3 000 MHz dans le mode UHF et apte à émettre ou recevoir un signal ayant une fréquence comprise entre 3 MHz et 30 MHz dans le mode HF, l'étiquette (18) comprenant en outre une mémoire (22) comprenant des informations,
la puce étant apte à communiquer selon le mode UHF et le mode HF,
dans lequel, dans le mode UHF, le lecteur UHF (108) présentant une portée donnée est apte à lire l'ensemble des étiquettes (18) présentes dans la portée, et
dans lequel, dans le mode HF, au moins une information privée est accessible par le lecteur HF (103), ladite information privée étant non accessible par le mode UHF, et dans lequel, dans le mode HF, le lecteur HF (103) est apte à lire une étiquette (18).

## Patentansprüche

1. Verfahren zum Verwalten mindestens eines einen biologischen Stoff enthaltenden Behälters (12), einen Schritt des Bereitstellens mindestens eines Behälters (12) umfassend, wobei jeder Behälter (12) mit einem Transponder (18) versehen ist und der Transponder (18) ein Chip zur drahtlosen Kommunikation ist, der geeignet ist, gemäß einer ersten Funktionsweise, genannt UHF Modus, und gemäß einer zweiten Funktionsweise, genannt HF Modus, zu kommunizieren, wobei der Chip geeignet ist, ein Signal zu senden und zu empfangen, das eine Frequenz zwischen 300 MHz und 3000 MHz in dem UHF Modus aufweist, und geeignet ist, ein Signal zu senden und zu empfangen, das eine Frequenz zwischen 3 MHz und 30 MHz in dem HF Modus aufweist, wobei der Transponder (18) außerdem einen Informationen enthaltenden Speicher (22) aufweist, wobei der Chip geeignet ist, mit einem ersten Leser (14) gemäß dem UHF Modus und mit einem zweiten Leser (16) gemäß dem HF Modus zu kommunizieren,
wobei das Verfahren außerdem die folgenden Schritte umfasst:
- Lesen, genannt UHF Lesen, bei dem der erste, eine vorgegebene Reichweite aufweisende Leser (14) die Gesamtheit der in der Reichweite vorhandenen Transponder (18) liest, wobei die Transponder (18) mit dem ersten Leser (14) gemäß der UHF Modus kommunizieren, und
- Lesen, genannt HF Lesen, mindestens einer nicht durch das UHF Lesen zugänglichen privaten Information, bei dem der zweite Leser (16) einen Transponder (18) liest, wobei der Transponder (18) mit dem zweiten Leser (16) gemäß dem HF Modus kommuniziert.

2. Verwaltungsverfahren nach Anspruch 1, bei dem während des HF Lesens der zweite Leser (16) mit einem Mal einen einzigen Transponder (18) liest.

3. Verwaltungsverfahren nach Anspruch 1 oder 2, bei dem der Transponder (18) einen ersten Teil von Informationen während des UHF Lesens und einen zweiten Teil von Informationen während des HF Lesens kommuniziert, wobei der zweite Teil nicht in dem ersten Teil eingeschlossen ist.

4. Verwaltungsverfahren nach Anspruch 3, bei dem der erste Teil von Informationen keine Informationen bezüglich der Herkunft des biologischen Stoffes aufweist.

5. Verwaltungsverfahren nach einem beliebigen der Ansprüche 1 bis 4, bei dem die Kommunikation zwischen dem Chip und dem Leser (14, 16) gemäß dem HF Modus mit einem NFC Protokoll durchgeführt wird.

6. Verfahren zum Übertragen von Informationen eines Mutterbehälters (12M) an eine Mehrzahl von Töchterbehälter (12F), wobei das Verfahren die folgenden Schritte umfasst:
- Bereitstellen eines Mutterbehälters (12M), der eine biologischen Stoff enthält,
- Verwalten des Mutterbehälters durch Durchführen eines Verwaltungsverfahrens, angewandt auf den Mutterbehälter (12M), nach einem beliebigen der Ansprüche 1 bis 5 zum Erhalten von Informationen bezüglich des Mutterbehälters (12M),
- Zentrifugieren des Mutterbehälters (12M) durch eine mit einem UHF Leser versehene Zentrifuge, wobei der UHF Leser das Vorhandensein des Mutterbehälters (12M) bei dem Zentrifugieren durch Kommunikation mit dem Transponder (18) des Mutterbehälters (12M) gemäß dem UHF Modus detektiert,
- Trennen der Komponenten des biologischen Stoffes in eine Mehrzahl von Töchterbehälter (12F) und
- Einschreiben von Informationen bezüglich des Mutterbehälters (12M) und von Informationen bezüglich des Zentrifugierens in jeden Speicher (22) der Transponder (18) der Töchterbehälter (12F) mittels einer Kommunikation gemäß dem HF Modus.

7. Verfahren zum Verfolgen von Behältern (12) in einer Gehäuse (102) mit kontrollierter Atmosphäre, die folgenden Schritte umfassend:
- Bereitstellen eines Gehäuses (102) mit kontrollierter Atmosphäre, das eine Mehrzahl von Fächern (104) aufweist, die jeweils ein Innenvolumen (106) begrenzen, wobei das Gehäuse (102) mit mindestens einem UHF Leser (108) ausgerüstet ist und die Behälter (12) an verschiedenen festgelegten Stellen angeordnet sind,
- Verwalten der Behälter (12) durch Durchführen des auf die Behälter (12) angewandten Verfahrens nach einem beliebigen der Ansprüche 1 bis 5, das das Lesen der Gesamtheit der Transponder (18) der Behälter (12) in jedem Fach (104) durch den entsprechenden UHF Leser (108) umfasst, und
- Verfolgen einer Stelle für jeden in dem Gehäuse (102) vorhandenen Behälter (12), wobei die Stelle dem entsprechenden UHF Leser (108) entspricht.

8. Vorrichtung (10) zum Verwalten mindestens eines einen biologischen Stoff enthaltenden Behälters (12), wobei die Vorrichtung einen Leser (14, 16) und mindestens eines Behälters (12) umfasst und der Behälter (12) mit einem Transponder (18) versehen ist und der Transponder (18) ein Chip zur drahtlosen Kommunikation ist, der geeignet ist, gemäß einer ersten Funktionsweise, genannt UHF Modus, und gemäß einer zweiten Funktionsweise, genannt HF Modus, zu kommunizieren, wobei der Chip geeignet ist, ein Signal zu senden und zu empfangen, das eine Frequenz zwischen 300 MHz und 3000 MHz in dem UHF Modus aufweist, und geeignet ist, ein Signal zu senden und zu empfangen, das eine Frequenz zwischen 3 MHz und 30 MHz in dem HF Modus aufweist, wobei der Transponder (18) außerdem einen Informationen enthaltenden Speicher (22) aufweist, wobei der Chip geeignet ist, mit dem Leser gemäß dem UHF Modus und gemäß dem HF Modus (14, 16) zu kommunizieren,
bei der der Transponder (18) geeignet ist, mit dem Leser (14, 16) einen ersten Teil von Informationen gemäß dem UHF Modus und einen zweiten Teil von Informationen, der mindestens eine private, nicht bei dem UHF Lesen zugängliche Information enthält, gemäß dem HF Modus zu kommunizieren.

9. Anordnung (100), die einen Leser, genannt HF Leser (103), der geeignet ist, ein Signal zu senden und empfangen, das eine Frequenz zwischen 3 MHz und 30 MHz aufweist, und ein Gehäuse (102) mit einer kontrollierten Atmosphäre umfasst, das eine Mehrzahl von jeweils ein Innenvolumen (106) begrenzenden Fächer (104) aufweist, wobei jedes Fach (104) mit einem Leser, genannt UHF Leser (108), ausgerüstet ist, der geeignet ist, ein Signal zu senden und zu empfangen, das eine Frequenz zwischen 300 MHz und 3000 MHz aufweist, wobei die Behälter (12) an verschiedenen festgelegten Stellen der Gehäuse (102) angeordnet sind,
wobei jeder Behälter (12) mit einem Transponder (18) versehen ist und der Transponder (18) ein Chip zur drahtlosen Kommunikation ist, der geeignet ist, gemäß einer ersten Funktionsweise, genannt UHF Modus, und gemäß einer zweiten Funktionsweise, genannt HF Modus, zu kommunizieren, wobei der Chip geeignet ist, ein Signal zu senden und zu empfangen, das eine Frequenz zwischen 300 MHz und 3000 MHz in dem UHF Modus aufweist, und geeignet ist, ein Signal zu senden und zu empfangen, das eine Frequenz zwischen 3 MHz und 30 MHz in dem HF Modus aufweist, wobei der Transponder (18) außerdem einen Informationen enthaltenden Speicher (22) aufweist,
wobei der Chip geeignet ist, gemäß dem UHF Modus und dem HF Modus zu kommunizieren,
bei der in dem UHF Modus der UHF Leser (108), der eine gegebene Reichweite aufweist, geeignet ist, die Gesamtheit der in der Reichweite vorhandenen Transponder (18) zu lesen, und
bei der in dem HF Modus mindestens eine private Information durch den HF Leser (103) zugänglich ist, wobei die private Information nicht von dem UHF Modus zugänglich ist und
bei der in dem HF Modus der HF Leser (103) geeignet ist, einen Transponder (18) zu lesen.

## Claims

1. A method for managing at least one container (12) containing a biological product, comprising a step of providing at least one container (12), each container (12) being provided with a tag (18), the tag (18) being a wireless communication chip capable of communicating according to a first operating mode called UHF mode and according to a second operating mode called HF mode, the chip being capable of emitting or receiving a signal having a frequency between 300 MHz and 3000 MHz in the UHF mode and capable of emitting or receiving a signal having a frequency between 3 MHz and 30 MHz in the HF mode, the tag (18) further comprising a memory (22) comprising information, the chip being capable of communicating with a first reader (14) according to the UHF mode and with a second reader according to the HF mode,
the method further comprising the following steps:
- UHF reading, in which the first reader (14) having a given range reads all of the tags (18) present within the range, the tags (18) communicating with the first reader (14) according to the UHF mode, and
- HF reading of at least a private piece of information non reachable with UHF reading, in which the second reader (14) reads a single tag (18), the tag (18) communicating with the second reader (16) according to the HF mode.

2. The management method according to claim 1, wherein, during the HF reading, the second reader (14) reads a single tag (18) at a time.

3. The management method according to claim 1 or 2, wherein the tag (18) communicates a first part of the information during the UHF reading and a second part of the information during the HF reading, the second part not being included in the first part.

4. The management method according to claim 3, wherein the first part of the information does not contain information relative to the origin of the biological product.

5. The management method according to any one of claims 1 to 4, wherein the communication between the chip and the reader (14, 16) according to the HF mode is implemented with the NFC protocol.

6. A method for transmitting information from a parent container (12M) to a plurality of daughter containers (12F), the method comprising the following steps:
- providing a parent container (12M) containing a biological product,
- managing the parent container by carrying out a management method applied to the parent container (12M) according to any one of claims 1 to 5 to obtain information relative to the parent container (12M),
- centrifuging the parent container (12M) using a centrifuge provided with the UHF reader, the UHF reader detecting the presence of the parent container (12M) during the centrifugation by communication with the tag (18) of the parent container (12M) according to the UHF mode,
- separating components of the biological product into a plurality of daughter containers (12F), and
- writing, on each memory (22) of the tags (18) of the daughter containers (12F), information relative to the parent container (12M) and information relative to the centrifugation owing to communication according to the HF mode.

7. A method for tracking containers (12) in an enclosure (102) with a controlled atmosphere, comprising the following steps:
- providing a controlled-atmosphere enclosure (102) having a plurality of compartments (104) each delimiting an inner volume (106), the enclosure (102) being equipped with at least one UHF reader (108), containers (12) being placed in various determined locations,
- managing the containers (12) by carrying out the management method applied to the containers (12) according to any one of claims 1 to 5, comprising reading all of the tags (18) of the containers (12) in each compartment (104) using the corresponding UHF reader (108), and
- tracking a location for each container (12) present in the enclosure (102), the location corresponding to the corresponding UHF reader (108).

8. A device (10) for managing at least one container (12) containing a biological product, the device comprising a reader (14, 16) and at least one container (12), the container (12) being provided with a tag (18), the tag (18) being a wireless communication chip capable of communicating according to a first operating mode called UHF mode and according to a second operating mode called HF mode, the chip being capable of transmitting or receiving a signal having a frequency of between 300 MHz and 3000 MHz in the UHF mode and capable of transmitting or receiving a signal having a frequency of between 3 MHz and 30 MHz in the HF mode, the tag (18) further comprising a memory (22) comprising information, the chip being capable of communicating with the reader according to the UHF mode or the HF mode (14, 16),
wherein the tag (18) is capable of communicating with the reader (14, 16) a first part of the information according to the UHF mode and a second part of the information comprising at least a piece of private information not reachable by UHF reading according to the HF mode.

9. An assembly (100) comprising a so-called HF reader (103), capable of receiving a signal having a frequency of between 3 MHz and 30 MHz, and a controlled-atmosphere enclosure (102) having a plurality of compartments (104) each delimiting an inner volume (106), each compartment (104) being equipped with a so-called UHF reader (108) capable of receiving a signal having a frequency of between 300 MHz and 3000 MHz, containers (12) being placed in various determined locations of the enclosure (102),
each container (12) being provided with a tag (18), the tag (18) being a wireless communication chip capable of communicating according to a first operating mode called UHF mode and according to a second operating mode called HF mode, the chip being capable of emitting or receiving a signal having a frequency between 300 MHz and 3000 MHz in the UHF mode and capable of emitting or receiving a signal having a frequency between 3 MHz and 30 MHz in the HF mode, the tag (18) further comprising a memory (22) comprising information,
the chip being capable of communicating according to the UHF mode and the HF mode,
wherein, in the UHF mode, the UHF reader (108) having a given range is adapted to read all the tags (18) within the given range, and
wherein, in the HF mode, at least one piece of private information is reachable by the HF reader (103), said piece of private information being non reachable by the UHF mode, and wherein, in the HF mode, the HF reader (103) is adapted to read a tag (18).
